# EUROPEAN PATENT APPLICATION

(11) **EP 4 838 536 A1**
(43) Date of publication of application: **11.11.2026**
(21) Application number: 25175245.7
(22) Date of filing: 09.05.2025
(51) Int. Cl.: G16H 15/00

(54) **ARTIFICIAL INTELLIGENCE SYSTEM ARCHITECTURE FOR GENERATING AND APPLYING CONFIDENCE METRICS IN AUTOMATIC ECG REPORTING**

(71) Applicant: Koninklijke Philips N.V., 5656 AE Eindhoven (NL)
(72) Inventor: PIJL, Marten, Eindhoven (NL); MAMPRIN, Marco, Eindhoven (NL)
(74) Representative: Philips Intellectual Property & Standards

(57) **Abstract**

Provided herein are artificial intelligence (AI) architectures for automatic electrocardiogram (ECG) reporting, and particularly systems and methods having integrated reporting confidence guardrails. As described, the systems and methods involve the use of a validation module to analyze AI-generated ECG reports, which generates a confidence level associated with the AI-generated reports. Based on the confidence level generated, the AI-generated report may be escalated in several different manners, such as to a trained technician for further review and analysis or directly to a healthcare provider for diagnosis, among others.

## Description

### Field of the Disclosure

The present disclosure relates generally to artificial intelligence architectures for automatic electrocardiogram (ECG) reporting, and more specifically to artificial intelligence architectures having integrated confidence guardrails.

### Background

Electrocardiograms (ECG) are recorded daily for numerous individuals to monitor heart abnormalities. These recordings can be conducted at hospitals, general practitioner offices, with Holter devices, or wearables such as the Apple Watch. Frequently, these recordings are prescribed for the purpose of diagnosing or treating cardiac conditions. Once recorded, the ECG data is analyzed to identify any abnormalities. This task is usually performed manually by an ECG technician who interprets the signals and creates a report for the cardiologist. This process can be time-consuming, particularly for long recordings from Holter devices, which can last weeks or months.

Recently, technicians are increasingly relying on artificial intelligence (AI) to pinpoint pertinent signal areas or detect cardiac abnormalities, thereby expediting the process. Nevertheless, the compilation of results into a comprehensive report continues to be labor-intensive, particularly in instances where there are minimal or no significant cardiac events. With recent advances in generative AI and large language models (LLMs), automated report generation is now more feasible. However, generative AI does have its own downsides, such as the tendency to 'hallucinate', i.e., make up statements that do not have any factual basis. In addition, generative AI is ill-equipped to handle serious or complex cases, where the intervention of a trained technician is necessary. Therefore, generative AI cannot be trusted to safely generate final clinical reports.

### Summary of the Disclosure

As mentioned above, while generative AI has the potential to improve patient monitoring by increasing the speed and accuracy of ECG monitoring, thereby providing actionable information to the relevant stakeholders more quickly and efficiently, these AI tools cannot be fully relied upon due to inconsistencies, inaccuracies, and the like. Therefore, described herein are integrated AI architectures that enable evaluation of AI-generated ECG reports to determine whether a report can be trusted and to determine whether intervention is required.

According to one embodiment of the present disclosure, an electrocardiogram system is provided. The electrocardiogram system can include a processing component comprising one or more computer processors and a computer-readable memory storing instructions that, when executed by the one or more computer processors, cause the electrocardiogram system to: (1) analyze, using an analysis module of the processing component, electrocardiogram data collected from an individual; (2) generate, using a report generator of the processing component, an electrocardiogram report based on the analysis of the electrocardiogram data, wherein the report generator comprises an artificial intelligence algorithm trained to receive analyses of electrocardiogram data and generate electrocardiogram reports based thereon; (3) generate, using a validation module, a confidence level associated with the electrocardiogram report; and (4) escalate, using a decision module, the electrocardiogram report based on the confidence level generated.

In an aspect, the electrocardiogram system can further include a wearable component having one or more physiological sensors configured to measure electrical activity of the individual's heart.

In an aspect, the electrocardiogram system can be further configured to: generate, via the wearable component, the electrocardiogram data for the individual; and communicate the electrocardiogram data from the wearable component to the processing component.

In an aspect, the validation module can include a content validator configured to receive the electrocardiogram report, assess one or more substantive portions of the electrocardiogram report, and output one or more sub-confidence levels associated with the one or more substantive portions of the electrocardiogram report. Further, the validation module can also include a structure validator configured to receive the electrocardiogram report, assess one or more structural aspects of the electrocardiogram report, and output one or more sub-confidence levels associated with the one or more structural aspects of the electrocardiogram report.

In an aspect, the confidence level associated with the electrocardiogram report may be generated based on the one or more sub-confidence levels associated with the one or more substantive portions of the electrocardiogram report and/or on the one or more sub-confidence levels associated with the one or more structural aspects of the electrocardiogram report.

In an aspect, the content validator may be further configured to receive medical history information for the individual from a medical history database and assess the one or more substantive portions of the electrocardiogram report based on the received medical history information.

In an aspect, the structure validator may be further configured to receive reporting knowledge information from a reporting knowledge database and assess the one or more structural aspects of the electrocardiogram report based on the received reporting knowledge information.

According to another embodiment of the present disclosure, a method of generating an electrocardiogram report is provided. The method may include: (1) analyzing, using an analysis module of a processing component, electrocardiogram data collected from an individual; (2) generating, using a report generator of the processing component, an electrocardiogram report based on the automatic analysis of the electrocardiogram data, wherein the report generator comprises an artificial intelligence algorithm trained to receive analyses of electrocardiogram data and generate electrocardiogram reports based thereon; (3) generating, using a validation module, a confidence level associated with the electrocardiogram report; and (4) escalating, using a decision module, the electrocardiogram report based on the confidence level generated.

In an aspect, the method can further include: generating, via a wearable component, the electrocardiogram data for the individual; and communicating the electrocardiogram data from the wearable component to the processing component.

In an aspect, generating the confidence level associated with the electrocardiogram report can include: validating, using a validation module of the processing component, one or more substantive portions of the electrocardiogram report and/or one or more structural aspects of the electrocardiogram report. The confidence level associated with the electrocardiogram report may be generated based on one or more outputs of the validation module.

In an aspect, validating the electrocardiogram report can include: (i) assessing the one or more substantive portions of the electrocardiogram report using a content validator to output one or more sub-confidence levels associated with the one or more substantive portions of the electrocardiogram report; and/or (ii) assessing the one or more structural aspects of the electrocardiogram report using a structure validator to output one or more sub-confidence levels associated with the one or more structural aspects of the electrocardiogram report.

In an aspect, the method may further include: receiving, from a medical history database, medical history information for the individual. The one or more substantive portions of the electrocardiogram report may be assessed based on the received medical history information using the content validator.

In an aspect, the method may further include: receiving, reporting knowledge information from a reporting knowledge database. The one or more structural aspects of the electrocardiogram report may be assessed based on the received reporting knowledge information using the structure validator.

According to yet another embodiment of the present disclosure, a computer program product comprising a non-transitory computer-readable memory is provided. The non-transitory computer-readable memory may store instructions that, when executed by one or more computer processors, cause the one or more processors to: analyze, using an analysis module, electrocardiogram data collected from an individual; generate, using a report generator, an electrocardiogram report based on the automatic analysis of the electrocardiogram data, wherein the report generator comprises an artificial intelligence algorithm trained to receive analyses of electrocardiogram data and generate electrocardiogram reports based thereon; generate, using a validation module, a confidence level associated with the electrocardiogram report; and escalate, using a decision module, the electrocardiogram report based on the confidence level generated.

In an aspect, the validation module may include a content validator configured to receive the electrocardiogram report, assess one or more substantive portions of the electrocardiogram report, and output one or more sub-confidence levels associated with the one or more substantive portions of the electrocardiogram report. The validation module may also include a structure validator configured to receive the electrocardiogram report, assess one or more structural aspects of the electrocardiogram report, and output one or more sub-confidence levels associated with the one or more structural aspects of the electrocardiogram report.

These and other aspects of the various embodiments will be apparent from and elucidated with reference to the embodiments described hereinafter.

### Brief Description of the Drawings

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
FIG. 1 is a workflow diagram for an electrocardiogram monitoring system illustrated in accordance with aspects of the present disclosure.
FIG. 2 is a functional block diagram of the analysis, validation, and decision components of an electrocardiogram monitoring system illustrated in accordance with aspects of the present disclosure.
FIG. 3 is a functional block diagram of a processing component of an electrocardiogram monitoring system illustrated in accordance with aspects of the present disclosure.
FIG. 4 is a flowchart illustrating a method of enhancing electrocardiogram reports generated through artificial intelligence in accordance with aspects of the present disclosure.

### Detailed Description of Embodiments

As mentioned above, generating ECG reports historically involves substantial manual effort. For large organizations that process hundreds or thousands of results daily, this leads to considerable overhead and expense. Additionally, many heart monitor users are healthy individuals who produce normal results, making the completion of these reports a trivial yet time-consuming task. With recent advances in generative AI, reports can be automatically generated. However, generative AI is prone to errors such as hallucinations. In addition, generative AI is ill-equipped to handle serious or complex cases, where the intervention of a trained technician is necessary.

Thus, in accordance with various aspects of the present disclosure, artificial intelligence system architectures and methods related to the generation of ECG reports are described, wherein said system architectures and methods employ methods to estimate confidence levels based on the particular outcomes of the assessment and automatic review of the AI-generated report. In accordance with certain aspects of the present disclosure, the AI-generated report may be accepted in whole or in part, rejected in whole or in part, or escalated to a human technician depending on the estimated confidence level.

The systems and methods described herein address a number of technical challenges in the healthcare field and specifically the field of patient monitoring. For example, according to certain aspects of the present disclosure, the systems and methods can reduce the time it takes physicians to make diagnoses, allows technicians to focus their time on the most relevant / critical cases, improves accuracy and reduces false negatives in ECG reviews, reduces the cost of life-saving care, and improves ECG monitoring efficiency and productivity. Overall, the systems and methods described herein improve patient and staff experiences, lead to better health outcomes, and lower the cost of important healthcare.

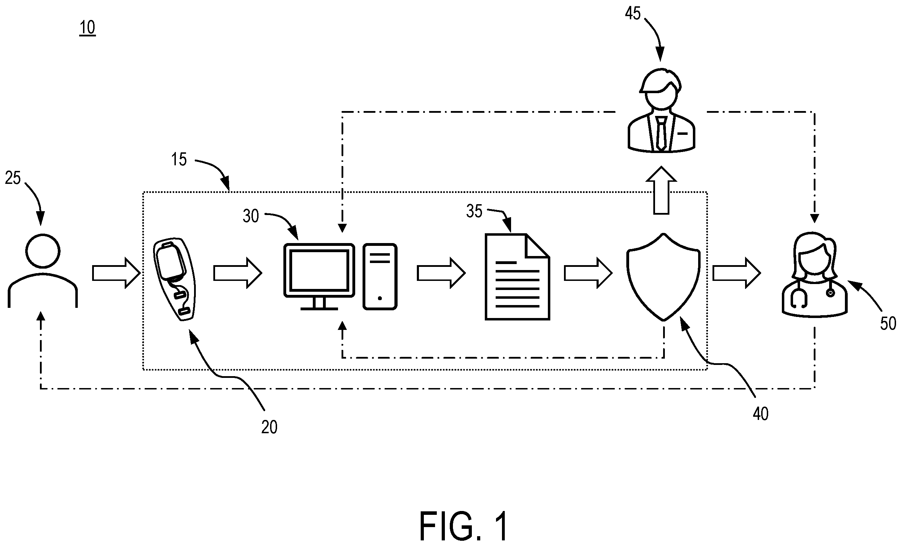

With reference to FIG. 1, an exemplary workflow 10 for utilizing a confidence estimation and artificial intelligence architectures having integrated confidence guardrails is illustrated according to various aspects of the present disclosure. As shown, the workflow 10 can include an artificial intelligence-based system 15 comprising one or more medical devices 20 configured to measure physiological signals of an individual 25, e.g., electrical signals associated with cardiac activity of the individual 25. In particular embodiments, the medical device 20 may be a wearable medical device configured to measure and record electrocardiogram (ECG) data for the individual 25 over extended periods of time, such as weeks to months. As such, it is practically impossible for a healthcare professional to review the hundreds of hours of ECG data containing hundreds of thousands or millions of datapoints. In particular embodiments, the medical device 20 can be configured especially for outpatient use, such as the Philips Holter system, the Philips Mobile Cardiac Telemetry monitor, and/or the like.

The artificial intelligence-based system 15 can include a computing device 30 that receives the ECG data from the one or more medical devices 20. As described in more detail below, the computing device 30 can include one or more computer processors and a computer-readable memory having instructions that, when executed by the one or more computer processors, executes an analysis module that causes the computing device 30 to: (i) analyze the ECG data received from one or more medical devices 20; and (ii) generate a report 35 using an AI model based on the analysis of the ECG data.

In preferred embodiments, the artificial intelligence-based system 15 further includes an escalation review component 40 that is configured to receive the AI-generated report 35 and apply a content validator and a structure validator to the AI-generated report 35 in order to determine a confidence assessment associated with the AI-generated report 35. The escalation review component 40 may further include a decision module that determines how to route the AI-generated report 35 based on the confidence assessment determined for the AI-generated report 35. For example, in particular embodiments, the escalation review component 40 may direct the AI-generated report to a physician or other healthcare professional 45 responsible for the care of the individual 25, to an ECG technician 50, and/or back to the computing device 30 as shown in FIG. 1.

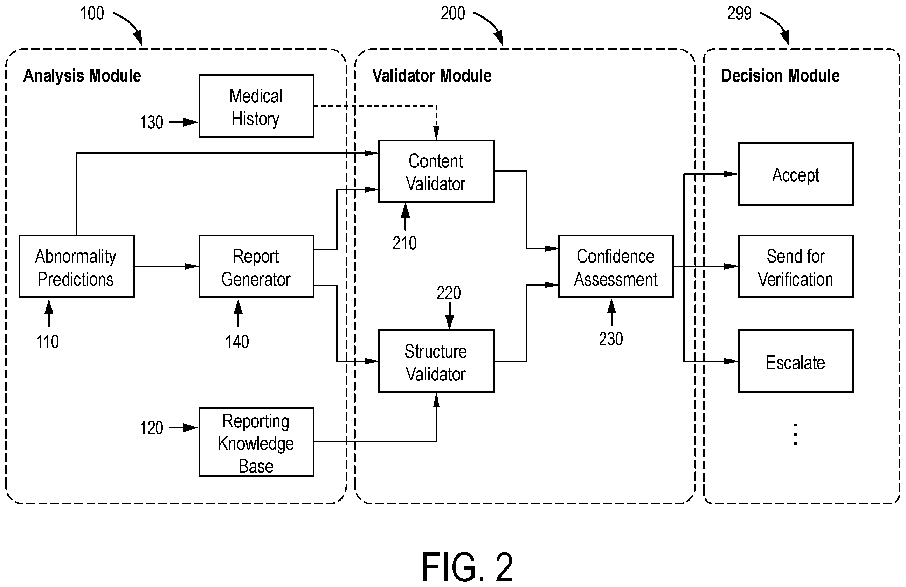

In embodiments, the escalation review component 40 may be executed by the computing device 40. However, it is also contemplated that the escalation review component 40 may be executed by a second computing device similar to computing device 30, which may be located remotely from the medical devices 20 and/or the computing device 30. In particular embodiments, the escalation review component 40 can include at least one of a validator module 200 and/or a decision module 299, as shown in FIG. 2.

More specifically, with reference to FIG. 2, the features of the analysis module 100, the validator module 200, and the decision module 299 are illustrated in more detail. In particular, the analysis module 100 can include an abnormality prediction component 110, a reporting knowledge database 120, and a medical history database 130, which can be used by a report generator 140 to produce the AI-generated report 35.

In particular embodiments, the abnormality prediction component 110 can be configured to identify and/or predict certain health conditions based on at least the ECG data received from the medical device 20. In embodiments, the abnormality prediction component 110 may be configured to automatically perform this analysis upon receiving ECG data for an individual 25, i.e., without requiring human intervention. The abnormality predictions can represent the assessment of the received ECG data in terms of which cardiac abnormalities are present in the ECG data. In some embodiments, the abnormality predictions may be produced through AI-based methods like deep learning, but may also be derived by applying a set of rules-based conditions to the received ECG data.

In embodiments, the reporting knowledge database 120 can store examples of previous reports, or additional context such as one or more report templates or technician instructions, as well as information such as possible report styles and/or the like. In particular embodiments, the report knowledge database 120 may include prompts that can be used by the report generator 140 to produce and/or refine the AI-generated report 35. In embodiments, the reporting knowledge database 120 may not include personally identifiable information (PII) or protected health information (PHI).

In embodiments, the report generator 140 can use the ECG data and the abnormality predictions to generate a physician report 35 using one or more AI algorithms, including but not limited to one or more generative AI models. In particular embodiments, the report generator 140 may use the ECG data and the abnormality predictions to automatically generate the report 35 upon receiving these inputs and/or an instruction to proceed to the reporting step. That is, the report 35 may be automatically generated with little or no human intervention. In some embodiments, the report generator 140 may be implemented as a large language model (LLM) or a similar generative AI technique. In embodiments, the AI model of the report generator 140 may be a pre-trained model that is finetuned with report knowledge, or may otherwise reference report knowledge from an external data source, such as the report knowledge database 120. In some embodiments, the report generator 140 may utilize a retrieval augmented generation (RAG) approach to produce the AI-generated report 35 in light of any specifications provided report knowledge database 120.

Once an AI-generated report 35 has been generated, the validator module 200 automatically evaluates the AI-generated report 35 based on certain criteria and generates a confidence assessment 230 for the AI-generated report 35. As shown in the example of FIG. 2, the validator module 200 can include at least a content validator 210 and a structure validator 220.

As described herein, the content validator 210 may be configured to assess the report 35 based on its content, and in particular, provide a measure of the confidence in the accuracy of the report 35 as well as the severity of a potential inaccuracy. In embodiments, the content validator 210 may itself be a separate, trained LLM or other type of AI model, such as a linear regression or gradient boosting model, or a combination of different models.

In particular aspects, the content assessment may be derived from the original models used to generate the report 35 and/or used to provide the abnormality predictions. For example, deep learning models for abnormality prediction can be configured (i.e., trained) to provide a confidence score as part of the output of the network. LLMs can also provide metrics to indicate the level of confidence in the response.

In further embodiments, the content validator 210 may utilize a severity metric, which can be derived based on the predicted abnormalities combined with medical domain knowledge provided by a medical history database 130 containing medical history information for the individual 25. For example, inaccuracies in a report 35 concerning an abnormality with no medical consequences would be more acceptable than for abnormalities with potential life-threatening consequences. In the latter case, the content validator 210 may provide extra scrutiny regardless of the estimated confidence in the generated report 35.

Similarly, if medical information is available from a medical history database 130, this information can also be used (in combination with other data such as the predicted abnormalities) to estimate severity. Medical information can be in the form of electronic health records (EHR), but may also include a patient diary, or symptoms recorded by the patient during use of the heart monitor.

In some embodiments, the content validator 210 may utilize a logical or probabilistic assessment to determine potential uncertainty in the reported outcomes. For example, if two cardiac abnormalities rarely occur together, but are both listed as predicted, this may be an indication that the predictions are uncertain about which cardiac abnormality the patient is suffering from. Such a method can be implemented using, for example, a rule-based system.

In still further embodiments, the content validator 210 may utilize a quality metric associated with the raw ECG signal to determine a confidence measure associated with the AI-generated report 35. For example, a noisy signal will be harder to interpret by the report generator 140, and therefore, the content validator 210 may analyze the raw ECG signal for noise. There are various ways to estimate the level of noise in a signal, such as by analyzing the frequency spectrum of the signal.

Thus, based on these factors, for example, the content validator 210 may produce a confidence measure of the report 35 based on its content. In embodiments, the validation module 200 may also include a structure validator 220 that is configured to determine whether the report 35 contains the necessary elements and formatting, rather than the contents of the report itself. This can be implemented as yet another LLM, but may also receive additional information from a reporting knowledge database 120, including information about report templates, a set of instructions for filling out such a report 35, and/or using information from a technician manual or industry guidelines.

In particular embodiments, the structure validator 220 may: (i) check the report 35 for the presence / absence of one or more sections or report features based on a list of components (e.g., a summary section, inclusion of ECG strips of detected abnormalities, and so on); (ii) visually inspect the report 35 using a vision-based LLM to determine if all elements are in the right position on the report 35; and/or (iii) check the text of the generated report 35 for spelling or grammar errors and other stylistic errors, such as the text being too long (and should be condensed) or repeated phrases or sentences.

Based on the outputs of the content validator 210 and the structure validator 220, a confidence assessment component 230 may then produce a confidence measure associated with the AI-generated report 35.

In some embodiments, a confidence interval may be provided to the report generator 140 for each cardiac abnormality prediction in order to allow the content validator 210 and the structure validator 220 to inherit appropriate confidence assessments with the main findings of the ECG data. At that stage, the validation module 200 will retain all the relevant predictions and their confidence in order to finalize the confidence assessment.

In embodiments, the confidence assessment component 230 may provide augmentations to the ECG data recorded for the individual 25 and determine what effect these augmentations have on the model outputs, e.g., the outputs of the analysis module 100. As described herein, the augmentations refer to subtle changes to the original ECG signal, e.g., by translating or scaling the signal. In embodiments, if the analysis module output (e.g., the report conclusions, content, structure, etc.) is stable across different augmentations, then the confidence assessment component 230 can assign a high confidence to a particular abnormality prediction and/or the report 35 overall.

In other / further embodiments, the confidence assessment component 230 may include an adversarial network (AN) and/or generative adversarial network (GAN) configured to determine whether the different abnormality predictions have high or low confidence. Inputs can be embeddings and predictions, and the output is a confidence measure. The AN / GAN can be trained to estimate confidence of the predictions and can be trained separately from the main architecture 10.

In still further embodiments, the confidence assessment component 230 may use a more specialized and dedicated, and maybe also more computationally intensive, network to confirm the prediction when uncertainty is detected. In embodiments, the dedicated network may analyze one output at the time.

In some embodiments, an ensemble approach using multiple techniques can be adopted, and an ensemble of ML/DL model can be used. If the predictions are stable across multiple ML/DL techniques, then the confidence assessment component 230 may determine that the confidence is high because the results are shown to be robust across multiple inference algorithms. In specific embodiments, a Bayesian based DL neural network may be used, which can be trained to estimate confidence.

As mentioned above, the escalation review component 40 may also include a decision module 299 that is configured to change how the AI-generated report 35 is handled based on the determined confidence level. For example, in some embodiments, if the confidence is high enough (e.g., exceeds a predetermined or dynamic upper threshold), the report 35 may be accepted as is, without further review by a technician 45. In some embodiments, the report 35 may require review by a technician 45 before being accepted (possibly with changes), if the confidence level does not meet a particular confidence threshold. In some embodiments, if confidence is low (e.g., falls below a predetermined or dynamic lower threshold), the AI-generated report 35 may be dismissed entirely, and the case is escalated to a technician 45 to be analysed fully.

Although certain routing decisions have been described, it is contemplated that other actions may be taken as well. For example, in some embodiments, the AI-generated report 35 may be routed back to the report generator 140 with instructions for specific changes. In embodiments, these instructions may be generated automatically based on the reporting knowledge database 120 and/or based on input received from an ECG technician 45.

Once an appropriate report 35 is generated (i.e., having a satisfactory confidence level), the report 35 may be communicated to a healthcare provider / professional 50 responsible for overseeing the care of the individual 25. In embodiments, the healthcare provider 50 may use the AI-generated report 35 to make a diagnosis with respect to the individual 25, provide a new treatment to the individual 25, and/or modify a current treatment plan for the individual 25.

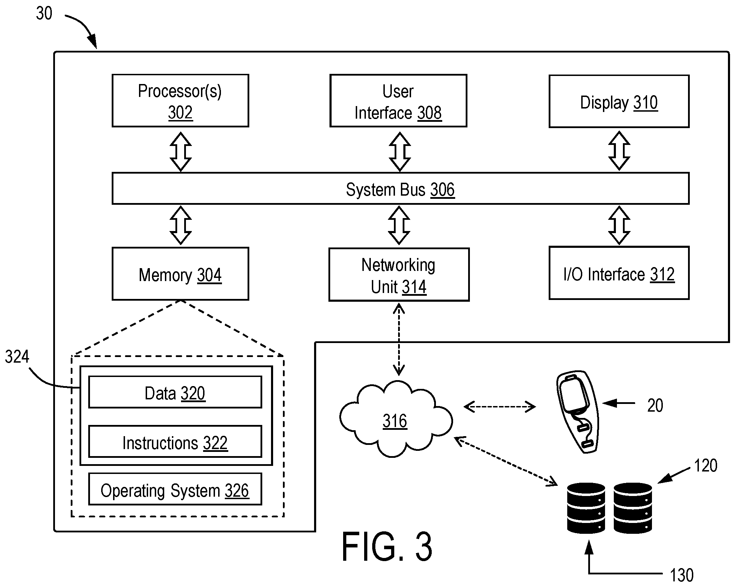

Turning now to FIG. 3, the computing device 30 of the artificial intelligence-based system 15 is illustrated according to further aspects of the present disclosure. As shown, the computing device 30 can include one or more processors 302 and a computer-readable memory 304 interconnected and/or in communication via a system bus 306 containing conductive circuit pathways through which instructions (e.g., machine-readable signals) may travel to effectuate communication, tasks, storage, and the like. The computing device 30 can be connected to a power source (not shown), which can include an internal power supply and/or an external power supply. In embodiments, the computing device 30 can also include one or more additional components, such as a user interface 308, a display 310, an input/output (I/O) interface 312, a networking unit 314, and the like, including combinations thereof. As shown, each of these components may be interconnected and/or in communication via the system bus 306, for example.

In embodiments, the one or more processors 302 can include one or more high-speed data processors adequate to execute the program components described herein and/or perform one or more operations of the methods described herein. The one or more processors 302 may include a microprocessor, a multi-core processor, a multithreaded processor, an ultra-low voltage processor, an embedded processor, and/or the like, including combinations thereof. The one or more processors 302 can include multiple processor cores on a single die and/or may be a part of a system on a chip (SoC) in which the processor 302 and other components are formed into a single integrated circuit, or a single package. That is, the one or more processors 302 may be a single processor, multiple independent processors, or multiple processor cores on a single die.

In embodiments, the user interface 308 may be configured to receive various forms of input from a user (e.g., technician 45 or healthcare provider 50, etc.) associated with the computing device 30. The user interface 308 can include, but is not limited to, one or more of a keyboard, keypad, trackpad, trackball(s), capacitive keyboard, controller (e.g., a gaming controller), computer mouse, computer stylus / pen, a voice input device, and/or the like, including combinations thereof.

In embodiments, the display device 310 may be configured to display information, including text, graphs, and/or the like. In particular embodiments, the display device 310 may be configured to display ECG strips from the ECG data, display AI-generated reports, and the like. The display device 310 can include, but is not limited to, a liquid crystal display (LCD), a light-emitting diode (LED) display, a touch screen or other touch-enabled display, a foldable display, a projection display, and so on, or combinations thereof.

In embodiments, the input/output (I/O) interface 312 may be configured to connect and/or enable communication with one or more peripheral devices (not shown), including but not limited to additional machine-readable memory devices, diagnostic equipment, and other attachable devices. The I/O interface 312 may include one or more I/O ports that provide a physical connection to the one or more peripheral devices. In some embodiments, the I/O interface 312 may include one or more serial ports.

In embodiments, the networking unit 314 may include one or more types of networking interfaces that facilitate wired and/or wireless communication between the computing device 30 and one or more external devices. That is, the networking unit 314 may operatively connect the computing device 30 to one or more types of communications networks 216, which can include a direction interconnection, the Internet, a local area network ("LAN"), a metropolitan area network ("MAN"), a wide area network ("WAN"), a wired or Ethernet connection, a wireless connection, a cellular network, and similar types of communications networks, including combinations thereof. In some embodiments, the computing device 30 may communicate with one or more medical devices 20, as well as one or more remote / cloud-based servers and/or cloud-based services, such as remote server 318, via the communications network 316. In particular embodiments, the remote servers may include the medical history database 130 and/or the reporting knowledge database 120, for example.

In embodiments, the memory 304 can be variously embodied in one or more forms of machine accessible and machine-readable memory. In some embodiments, the memory 304 includes a storage device (not shown), which can include, but is not limited to, a non-transitory storage medium, a magnetic disk storage, an optical disk storage, an array of storage devices, a solid-state memory device, and/or the like, as well as combinations thereof. The memory 204 may also include one or more other types of memory, such as dynamic random-access memory (DRAM), static random-access memory (SRAM), erasable programmable read-only memory (EPROM), electrically erasable programmable read-only memory (EEPROM), Flash memory, and/or the like, as well as combinations thereof. In embodiments, the memory 304 may include one or more types of transitory and/or non-transitory memory.

The computing device 30 can be configured by software components stored in the memory 304 to perform one or more processes of the methods described herein. More specifically, the memory 304 can be configured to store data / information 320 and computer-readable instructions 322 that, when executed by the one or more processors 302, causes the computing device 30 to generate an ECG report 35, determine a confidence level associated with the report 35, and route the ECG report 35 to one or more destinations for further actions based on the confidence level. Such data 320 and the computer-readable instructions 322 stored in the memory 304 may form a report confidence package 324 that may be incorporated into, loaded from, loaded onto, or otherwise operatively available to and from the computing device 30. Thus, in some embodiments, the report confidence package 324 and/or one or more individual software packages may be stored in a local storage device of the memory 304. However, in other embodiments, the report confidence package 324 and/or one or more individual software packages may be loaded onto and/or updated from a remote server or service via the communications network 316.

The computing device 30 may also include an operating system component 326, which may be stored in the memory 304. The operating system component 326 may be an executable program facilitating the operation of the computing device 30. Typically, the operating system component 326 can facilitate access of the I/O interface 312, network interface 314, the user interface 308, and the display 310, and can communicate or control other components of the computing device 30.

Accordingly, provided herein is a computer program product 324 comprising a non-transitory computer-readable storage medium 304 having stored thereon computer-readable instructions 322 that, when executed by one or more processors (such as processors 302), cause the one or more processors to perform one or more operations of the methods described below.

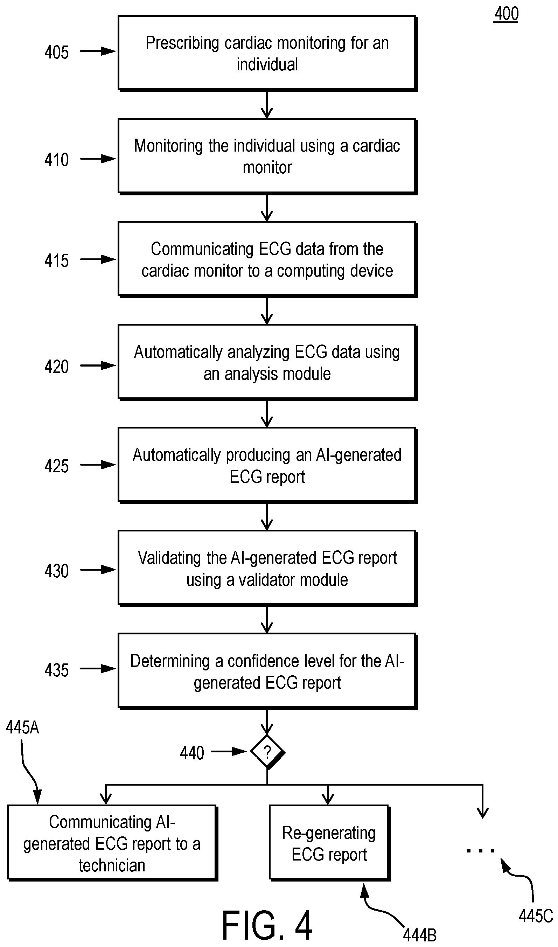

For example, with reference to FIG. 4, a method 400 for escalating AI-generated ECG reports is illustrated in accordance with certain aspects of the present disclosure. As shown, the method 400 can include: in a step 410, monitoring an individual 25 using a cardiac monitor 20; in a step 415, communicating ECG data recorded using the cardiac monitor 20 to a computing device 30; in a step 420, analyzing the ECG data using an analysis module 100; producing an AI-generated ECG report 35; in a step 430, validating the AI-generated ECG report 35 using a validator module 200; in a step 435, determining a confidence level for the AI-generated ECG report 35; in a step 440, determining how to escalate the AI-generated ECG report 35 based on the determined confidence level. In certain embodiments, the method 400 can also include, in a step 405, prescribing cardiac monitoring for the individual 25; and/or in a step 445, taking an action with respect to the AI-generated ECG report 35 based on the determined confidence level.

More specifically, in the step 410, the method 400 can include collecting ECG data from one or more individuals 25 using one or more cardiac monitoring devices 20. In embodiments, the cardiac / ECG data may be collected over an extended period of time, such as at least one week. In some embodiments, the data may be collected for several weeks, such as up to one month.

In the step 415, the method 400 can include communicating the ECG data to a computing device 30, which may be located remotely from the monitoring device 20. In embodiments, the step 415 may include transmitting the ECG data from the monitoring device 20 using a wired and/or wireless communications network, and then receiving the ECG data via the wired and/or wireless communications network at the computing device 30. In embodiments, the ECG data may be communicated periodically or sporadically, according to a particular schedule, based on network conditions and connectivity, and/or according to an amount of ECG data collected (e.g., once a certain amount of the on-board memory of the cardiac monitor 20 has been filled). In other words, the ECG data may be communicated one or more times over a particular monitoring period during which the individual 25 is being monitored.

In the step 420, the method 400 can include automatically analyzing the ECG data using an analysis module 100. In embodiments, the analysis module 100 can analyze the days, weeks, or months-worth of ECG data collected from the cardiac monitor 20. As described above, an abnormality prediction component 110 may analyze the ECG data to identify any potential abnormalities indicative of a medical condition of the individual 25.

In the step 425, the method 400 can then include automatically producing an AI-generated ECG report 35 using a report generator 140. In embodiments, the report generator 140 may include at least a second trained AI-based algorithm configured to write a report 35 based on at least the ECG data collected and the predicted abnormalities. In some embodiments, the report 35 may also include relevant information from the medical history of the individual 25, which may be retrieved from a medical history database 130.

In a step 430, the method 400 can then include validating the AI-generated ECG report 35 using a validator module 200. In particular embodiments, the step 430 may include validating the content of the ECG report 35 using a content validator 210 and/or validating the structure of the ECG report 35 using a structure validator 220, as described above.

**In** a step 435, the method 400 can then include determining a confidence level for the ECG report 35 based on the output of the content and structure validators 210, 220. In embodiments, the confidence level may be determined using a confidence assessment component 230, as described above.

**In** a step 435, the method 400 can then include determining how to escalate the ECG report 35 based on the determined confidence level. In embodiments, the step 435 can include using a decision module 299 to apply a set of rules based on the confidence level determined for the ECG report 35. For example, a rules-based function, *f*, like the one below may be applied: $f \left({c}_{i}\right) = \left\{\begin{matrix}Action 1 & {c}_{i} \leq {t}_{1} \\ Action 2 & {t}_{1} < {c}_{i} \leq {t}_{2} \\ ⋮ & ⋮ \\ Action N & {c}_{i} > {t}_{n}\end{matrix}\right.$ wherein *cᵢ* is the confidence level determined for an ECG report *i, t*₁, *t*₂, ... , *tₙ* are predetermined threshold levels, and *n* is an integer greater than 2.

However, it is contemplated that other techniques for determining how to escalate the ECG 35 report may be utilized. For example, in some embodiments, the confidence level (*cᵢ*) for an ECG report 35 may be a composite of multiple sub-confidence levels, e.g., *cᵢ =* {*p*₁ *p*₂ ··· *pₘ*}, where each sub-confidence level p corresponds to a confidence level of a specific abnormality prediction made based on the received ECG data. In such embodiments, the decision module 299 may account for prediction-specific confidences, such as by escalating the ECG report 35 if a confidence level for a high-risk abnormality is low even though the overall confidence level for the ECG report 35 is high.

Depending on the rules of the decision module 299, the method 400 can include, in one or more steps 445A, 445B, 445C, escalating the ECG report 35 to different destinations. For example, in a step 445A, the ECG report 35 may be escalated to a trained technician, e.g., by alerting a trained ECG technician of the need to review one or more portions of the ECG report 35 and communicated the ECG report 35 to the ECG technician, or otherwise making the ECG report 35 available to the technician (such as through a technician workflow portal). According to some embodiments, the ECG report 35 may be escalated by re-generating the ECG report 35, e.g., by communicating errors or issues with the ECG report 35 to the processing component 30 and repeating steps 420 and/or 425. According to further embodiments, in a step 445C, the ECG report 35 may be escalated to another destination, such as to a healthcare provider 50 who can use the ECG report 35 to prescribe a particular treatment or therapy, or to modify an existing treatment or therapy, for the individual 25.

It should be appreciated that all combinations of the foregoing concepts and additional concepts discussed in greater detail below (provided such concepts are not mutually inconsistent) are contemplated as being part of the inventive subject matter disclosed herein. **In** particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The terms "individual", "patient", and "subject" are used herein synonymously and interchangeably unless otherwise specified explicitly or implicitly by the context of its use. **In** various examples, these terms may refer to a human person, but can also refer to a non-human animal.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. An electrocardiogram system (10) comprising:
a processing component (30) comprising one or more computer processors (302) and a computer-readable memory (304) storing instructions (322) that, when executed by the one or more computer processors (302), cause the electrocardiogram system (10) to:
automatically analyze, using an analysis module (100) of the processing component (30), electrocardiogram data collected from an individual (25);
automatically generate, using a report generator (140) of the processing component (30), an electrocardiogram report (35) based on the automatic analysis of the electrocardiogram data, wherein the report generator (140) comprises an artificial intelligence algorithm trained to receive analyses of electrocardiogram data and generate electrocardiogram reports based thereon;
generate, using a validation module (200), a confidence level associated with the electrocardiogram report (35); and
escalate, using a decision module (299), the electrocardiogram report (35) based on the confidence level generated.

2. The electrocardiogram system (10) of claim 1, further comprising a wearable component (20), the wearable component (20) having one or more physiological sensors configured to measure electrical activity of an individual's heart.

3. The electrocardiogram system (10) of claim 2, wherein the computer-readable memory (304) stores instructions (322) that, when executed by the one or more computer processors (302), further cause the electrocardiogram system (10) to:
generate, via the wearable component (20), the electrocardiogram data for the individual (25); and
communicate the electrocardiogram data from the wearable component (20) to the processing component (30).

4. The electrocardiogram system (10) of claim 1, wherein the validation module (200) comprises a content validator (210) configured to receive the electrocardiogram report (35), assess one or more substantive portions of the electrocardiogram report (35), and output one or more sub-confidence levels associated with the one or more substantive portions of the electrocardiogram report (35), and
wherein the validation module (200) further comprises a structure validator (220) configured to receive the electrocardiogram report (35), assess one or more structural aspects of the electrocardiogram report (35), and output one or more sub-confidence levels associated with the one or more structural aspects of the electrocardiogram report (35).

5. The electrocardiogram system (10) of claim 4, wherein the confidence level associated with the electrocardiogram report (35) is generated based on the one or more sub-confidence levels associated with the one or more substantive portions of the electrocardiogram report (35) and/or on the one or more sub-confidence levels associated with the one or more structural aspects of the electrocardiogram report (35).

6. The electrocardiogram system (10) of claim 4, wherein the content validator (210) is further configured to receive medical history information for the individual (25) from a medical history database (130) and assess the one or more substantive portions of the electrocardiogram report (35) based on the received medical history information.

7. The electrocardiogram system (10) of claim 4, wherein the structure validator (220) is further configured to receive reporting knowledge information from a reporting knowledge database (120) and assess the one or more structural aspects of the electrocardiogram report (35) based on the received reporting knowledge information.

8. A method (400) of generating an electrocardiogram report, the method (400) comprising:
automatically analyzing (420), using an analysis module (100) of a processing component (30), electrocardiogram data collected from an individual (25);
automatically generating (425), using a report generator (140) of the processing component (30), an electrocardiogram report (35) based on the automatic analysis of the electrocardiogram data, wherein the report generator (140) comprises an artificial intelligence algorithm trained to receive analyses of electrocardiogram data and generate electrocardiogram reports based thereon;
generating (430, 435), using a validation module (200), a confidence level associated with the electrocardiogram report (35); and
escalating (440, 445), using a decision module (299), the electrocardiogram report (35) based on the confidence level generated.

9. The method (400) of claim 8, further comprising:
generating (410), via a wearable component (20), the electrocardiogram data for the individual (25); and
communicating (410) the electrocardiogram data from the wearable component (20) to the processing component (30).

10. The method (400) of claim 8, wherein generating the confidence level associated with the electrocardiogram report (35) comprises:
validating (430), using a validation module (200) of the processing component (30), one or more substantive portions of the electrocardiogram report (35) and/or one or more structural aspects of the electrocardiogram report (35);
wherein the confidence level associated with the electrocardiogram report (35) is generated based on one or more outputs of the validation module (200).

11. The method (400) of claim 10, wherein validating the electrocardiogram report (35) includes:
assessing the one or more substantive portions of the electrocardiogram report (35) using a content validator (210) to output one or more sub-confidence levels associated with the one or more substantive portions of the electrocardiogram report (35); and
assessing the one or more structural aspects of the electrocardiogram report (35) using a structure validator (220) to output one or more sub-confidence levels associated with the one or more structural aspects of the electrocardiogram report (35).

12. The method (400) of claim 11, further comprising:
receiving, from a medical history database (130), medical history information for the individual (25);
wherein the one or more substantive portions of the electrocardiogram report (35) are assessed based on the received medical history information using the content validator (210).

13. The method (400) of claim 11, further comprising:
receiving, reporting knowledge information from a reporting knowledge database (120);
wherein the one or more structural aspects of the electrocardiogram report (35) are assessed based on the received reporting knowledge information using the structure validator (220).

14. A computer program product (324) comprising a non-transitory computer-readable memory (304) storing instructions (322) that, when executed by one or more computer processors (302), cause the one or more processors (302) to:
automatically analyze, using an analysis module (100), electrocardiogram data collected from an individual (25);
automatically generate, using a report generator (140), an electrocardiogram report (35) based on the automatic analysis of the electrocardiogram data, wherein the report generator (140) comprises an artificial intelligence algorithm trained to receive analyses of electrocardiogram data and generate electrocardiogram reports based thereon;
generate, using a validation module (200), a confidence level associated with the electrocardiogram report (35); and
escalate, using a decision module (299), the electrocardiogram report (35) based on the confidence level generated.

15. The computer program product (324) of claim 14, wherein the validation module (200) comprises a content validator (210) configured to receive the electrocardiogram report (35), assess one or more substantive portions of the electrocardiogram report (35), and output one or more sub-confidence levels associated with the one or more substantive portions of the electrocardiogram report (35), and
wherein the validation module (200) further comprises a structure validator (220) configured to receive the electrocardiogram report (35), assess one or more structural aspects of the electrocardiogram report (35), and output one or more sub-confidence levels associated with the one or more structural aspects of the electrocardiogram report (35).
